# DEMANDE DE BREVET EUROPEEN

(11) **EP 1 430 878 A1**
(43) Date de publication de la demande: **23.06.2004**
(21) Numéro de dépôt: 03293128.9
(22) Date de dépôt: 12.12.2003
(51) Int. Cl.: A61K 7/135, A61K 7/13

(54) **Composition tinctoriale des fibres kératiniques comprenant au moins un composé ortho- ou alpha-dialdehyde et au moins un composé soufre**

(30) Priorité: 16.12.2002 FR 0215913
(71) Demandeur: L'OREAL, 75008 Paris (FR)
(72) Inventeur: Plos, Grégory, 75011 Paris (FR)
(74) Mandataire: Wattremez, Catherine

(57) **Abrégé**

L'invention a pour objet une composition tinctoriale des fibres kératiniques, en particulier des fibres kératiniques humaines telles que les cheveux, comprenant, dans un milieu de teinture approprié, au moins un composé ortho ou α-dialdéhyde et au moins un composé soufré. L'invention a aussi pour objet l'utilisation de cette composition pour la teinture et / ou l'éclaircissement optique des fibres kératiniques ainsi que le procédé de teinture mettant en oeuvre cette composition.

## Description

La présente invention a pour objet une composition tinctoriale des fibres kératiniques, en particulier des fibres kératiniques humaines telles que les cheveux, comprenant, dans un milieu de teinture approprié, au moins un composé ortho ou α-dialdéhyde et au moins un composé soufré.

Classiquement, l'éclaircissement des fibres kératiniques est obtenu par l'utilisation d'agents oxydants tels que les persels ou l'eau oxygénée, qui dégradent les pigments mélaniques naturels et / ou les pigments artificiels présents dans les cheveux. Ce procédé d'éclaircissement chimique est efficace en terme d'éclaircissement mais présente l'inconvénient d'être agressif pour les cheveux.

Le but de l'invention est de fournir de nouvelles compositions qui permettent d'obtenir un éclaircissement des fibres kératiniques et qui ne présentent pas les inconvénients de celles de l'art antérieur. En particulier, le but de la présente invention est de fournir un nouveau système de teinture qui présente à la fois les avantages de la ténacité en particulier aux shampooings répétés et du respect de la fibre capillaire.

Ce but est atteint avec la présente invention qui a pour objet une composition tinctoriale des fibres kératiniques comprenant, dans un milieu de teinture approprié :
- au moins un composé ortho ou α-dialdéhyde de formule (I) : 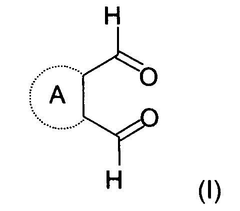
dans laquelle A est choisi parmi :
- un groupement mono ou polycarbocyclique, condensé ou non condensé, aromatique ou non aromatique, comprenant de 6 à 50 atomes de carbone ;
- un groupement mono ou polyhétérocyclique, condensé ou non condensé, aromatique ou non aromatique, comportant de 5 à 30 chaînons et contenant un ou plusieurs hétéroatomes choisis parmi l'azote, le soufre, l'oxygène et le phosphore ;
   le groupement A pouvant être substitué par des radicaux halogéno, alkyle en C₁-C₄, hydroxy, alcoxy en C₁-C₄, hydrogénocarbonyle, alkylcarbonyle en C₁-C₄, alkylthio en C₁-C₄, alkylcarboxy en C₁-C₄, nitro, sulfonato, ammonio, trialkylammonio en C₁-C₄, imidazolio, pyridinio, benzothiazolio ;
- au moins un composé soufré de formules (II) ou (III) :

   R-SH (II)

   R-S-S-R' (III)
dans lesquelles R et R', indépendamment l'un de l'autre, représentent un groupement comportant de 1 à 100 atomes de carbone et de préférence de 1 à 50, saturé ou insaturé, comportant éventuellement de 1 à 30 insaturations et de préférence de 1 à 10, ramifié ou non ramifié et pouvant comporter un ou plusieurs hétéroatomes choisis parmi l'azote, le soufre, l'oxygène et le phosphore, R et R' pouvant être substitués par un ou plusieurs radicaux choisis parmi les radicaux halogéno, alkyle en C₁-C₄, hydroxy, alcoxy en C₁-C₄, hydrogénocarbonyle, alkylcarbonyle en C₁-C₄, alkylthio en C₁-C₄, alkylcarboxy en C₁-C₄, amino, mono ou dialkylamino en C₁-C₄, mono ou dihydroxyalkylamino en C₁-C₄, nitro, sulfonato, ammonio, trialkylammonio en C₁-C₄, imidazolio, pyridinio, benzothiazolio.

La composition conforme à l'invention permet d'obtenir une coloration fluorescente tout en interagissant avec les acides aminés présents dans les cheveux. Il en résulte une grande ténacité de la coloration obtenue au cours du temps même si les cheveux subissent des lavages répétés.

Cette composition permet de plus d'obtenir une coloration des fibres kératiniques sans dégradation de celles-ci puisqu'elle peut être mise en oeuvre en l'absence d'agents oxydants. Avec cette composition, la teinture obtenue permet d'obtenir un éclaircissement optique de la fibre.

L'invention a aussi pour objet un procédé de teinture des fibres kératiniques, en particulier des fibres kératiniques humaines telles que les cheveux, utilisant cette composition, ainsi qu'un dispositif à plusieurs compartiments permettant de mettre en oeuvre ce procédé.

Un autre objet de l'invention est l'utilisation de la composition de la présente invention pour la teinture et / ou l'éclaircissement optique des fibres kératiniques, en particulier les fibres kératiniques humaines telles que les cheveux.

Au sens de la présente invention, on entend par éclaircissement optique un effet visuel d'éclaircissement des fibres kératiniques colorées, naturellement ou artificiellement, sans utiliser de composés détruisant les pigments colorés, naturels ou artificiels, présents dans les fibres kératiniques.

Dans le cadre de la présente invention, le terme "condensé" signifie au moins deux cycles accolés présentant au moins deux atomes communs.

Un groupement mono ou polycarbocyclique, condensé ou non condensé, aromatique ou non aromatique, comprenant de 6 à 50 atomes de carbone peut être par exemple un cycle benzène, naphtalène ou anthracène. Un groupement mono ou polyhétérocyclique, condensé ou non condensé, aromatique ou non aromatique, comportant de 5 à 30 chaînons et contenant un ou plusieurs hétéroatomes peut être par exemple un cycle thiofène, benzofuranne, benzothiofène, indole, bispyridine, benzopyrane, quinoline, pyrazole, pyridine, pyrrole, furanne, imidazole, benzimidazole. Le groupement polyhétérocyclique peut être condensé ou substitué par un ou plusieurs groupements carbocycliques.

On entend par radical alkyle (alk) un radical alkyle linéaire ou ramifié par exemple méthyle, éthyle, n-propyle, iso-propyle, butyle. Un radical alcoxy est un radical alk-O-, un radical alkylthio est un radical alk-S-, un radical alkylcarbonyle est un radical alk-CO-, un radical alkylcarboxy est un radical alk-COO-, un radical mono ou dialkylamino est un radical -N(alk)ₙ avec n = 1 ou 2, un radical mono ou dihydroxyalkylamino est un radical -N(alk-OH)ₘ avec m = 1 ou 2, dans chacune de ces définitions le radical alkyle ayant la définition donnée ci-dessus.

Un radical sulfonato est un radical -SO₃⁻. Un radical trialkylammonio est un radical (alk)₃N⁺- avec le radical alkyle ayant la définition donnée ci-dessus. Les radicaux imidazolio, pyridinio et benzothiazolio sont les radicaux cationiqués correspondant aux cations imidazolium, pyridinium et benzothiazolium.

Un groupement halogéno désigne un atome d'halogène choisi de préférence parmi le chlore, le brome et l'iode.

Par insaturations, on entend des doubles ou triples liaisons et celles-ci peuvent conduire à des structures aromatiques ou hétéroaromatiques.

On entend par acides aminés toute structure organique possédant une fonction acide carboxylique et une fonction amine terminales.

Selon un mode particulier de la composition de l'invention, le composé ortho ou α-dialdéhyde de formule (I) est un dérivé de l'orthophtalaldéhyde de formule (IV) : 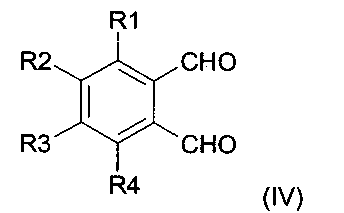 dans laquelle R₁, R₂, R₃ et R₄ représentent chacun indépendamment un atome d'hydrogène, un radical halogéno, alkyle en C₁₋₄, hydroxy, alcoxy en C₁-C₄, alkylthio en C₁-C₄, alkylcarboxy en C₁-C₄, nitro, sulfonato ou un groupement hétérocyclique non aromatique azoté comportant de 5 à 8 chaînons.

Les groupements R₁, R₂, R₃ et R₄ sont de préférence choisis parmi les groupements alkyle en C₁-C₄ et alcoxy en C₁-C₄.

A titre d'exemple, le dérivé de l'orthophtalaldéhyde de formule (IV) est choisi parmi l'orthophtalaldéhyde, le 4,5-diméthoxyphtalaldéhyde.

Selon un autre mode de réalisation particulier de la composition de l'invention, le composé ortho ou α-dialdéhyde de formule (I) est choisi parmi les dérivés du naphtalènedicarboxaldéhyde, de l'anthracènedicarboxaldéhyde et du thiofènedicarboxaldéhyde.

A titre d'exemple, le composé ortho ou α-dialdéhyde de formule (I) est choisi parmi le 1,2-naphtalènedicarboxaldéhyde, le 2,3-naphtalènedicarboxaldéhyde, le 2,3-anthracènedicarboxaldéhyde, le 2,3-thiofènedicarboxaldéhyde.

Selon un mode de réalisation particulier de la composition de l'invention, R et R' comprennent de 1 à 4 atomes de carbone.

Le composé soufré de formule (II) est de préférence choisi parmi l'éthanethiol, le 2-aminothiophénol, l'acide thioglycolique, la cystéamine. A titre d'exemple, le composé soufré de formule (III) est la cystamine.

La composition conforme à l'invention peut en outre contenir un ou plusieurs acides aminés choisis parmi les acides aminés naturels et les acides aminés de synthèse de forme D ou de forme L.

A titre d'exemple, le ou les acides aminés sont choisis parmi la glycine, l'alanine, la valine, la leucine, l'isoleucine, la méthionine, la phénylalanine, la proline, la sérine, la thréonine, la cystéine, l'asparagine, la glutamine, la tyrosine, l'histidine, la lysine, l'ornithine, l'arginine, l'acide aspartique, l'acide glutamique, le tryptophane.

La composition conforme à l'invention peut en outre contenir un ou plusieurs peptides et / ou une ou plusieurs protéines.

Les concentrations des différents composants de la composition de l'invention dépendent de l'intensité de coloration voulant être obtenue. Cependant, la concentration en composé ortho ou α-dialdéhyde de formule (I) est en général comprise entre 0,01 et 30% en poids, de préférence entre 0,05 et 20% en poids, rapporté au poids total de la composition, la concentration en composé soufré de formules (II) ou (III) est en général comprise entre 0,01 et 30% en poids, de préférence entre 0,05 et 20% en poids, rapporté au poids total de la composition et la concentration en acides aminés, lorsqu'ils sont présents, est en général comprise entre 0,01 et 20% en poids, de préférence entre 0,05 et 30% en poids, rapporté au poids total de la composition.

La composition conforme à l'invention comprend de préférence au moins un composé choisi parmi les agents tensioactifs, les polyols, les éthers de polyols et les mono-alcools aromatiques. La concentration individuelle en agents tensioactifs, polyols, éthers de polyols et mono-alcools aromatiques, lorsqu'ils sont présents, est en général comprise entre 0,1 et 20% en poids, de préférence entre 0,5 et 30% en poids, rapporté au poids total de la composition.

Le ou les agents tensioactifs peuvent être indifféremment choisis, seuls ou en mélanges, au sein des tensioactifs anioniques, amphotères, non ioniques, zwittérioniques et cationiques.

A titre d'exemple de tensioactifs anioniques utilisables, seuls ou en mélanges, dans le cadre de la présente invention, on peut citer notamment les sels, en particulier sels alcalins, notamment de sodium, sels d'ammonium, sels d'amines, sels d'aminoalcools ou sels de magnésium, des composés suivants : les alkylsulfates, les alkyléthersulfates, alkylamidoéthersulfates, alkylarylpolyéthersulfates, monoglycérides sulfates ; les alkylsulfonates, alkylphosphates, alkylamidesulfonates, alkylarylsulfonates, α-oléfine-sulfonates, paraffine-sulfonates ; les alkyl(C₆-C₂₄) sulfosuccinates, les alkyl(C₆-C₂₄) éthersulfosuccinates, les alkyl(C₆-C₂₄) amidesulfosuccinates ; les alkyl(C₆-C₂₄) sulfoacétates ; les acyl(C₆-C₂₄) sarcosinates et les acyl(C₆-C₂₄) glutamates. On peut également utiliser les esters d'alkyl(C₆-C₂₄)polyglycosides carboxyliques tels que les alkylglucoside citrates, les alkylpolyglycoside tartrate et les alkylpolyglycoside sulfosuccinates., les alkylsulfosuccinamates ; les acyliséthionates et les N-acyltaurates, le radical alkyle ou acyle de tous ces différents composés comportant de préférence de 12 à 20 atomes de carbone, et le radical aryl désignant de préférence un groupement phényle ou benzyle. Parmi les tensioactifs anioniques encore utilisables, on peut également citer les sels d'acides gras tels que les sels des acides oléique, ricinoléique, palmitique, stéarique, les acides d'huile de coprah ou d'huile de coprah hydrogénée ; les acyl-lactylates dont le radical acyle comporte 8 à 20 atomes de carbone. On peut également utiliser les acides d'alkyl D galactoside uroniques et leurs sels, les acides alkyl (C₆-C₂₄) éther carboxyliques polyoxyalkylénés, les acides alkyl(C₆-C₂₄)aryl éther carboxyliques polyoxyalkylénés, les acides alkyl(C₆-C₂₄) amido éther carboxyliques polyoxyalkylénés et leurs sels, en particulier ceux comportant de 2 à 50 groupements oxyde d'alkylène en particulier d'éthylène, et leurs mélanges.

Les agents tensioactifs non-ioniques sont, eux aussi, des composés bien connus en soi (voir notamment à cet égard "Handbook of Surfactants" par M.R. PORTER, éditions Blackie & Son (Glasgow and London), 1991, pp 116-178) et leur nature ne revêt pas, dans le cadre de la présente invention, de caractère critique. Ainsi, ils peuvent être notamment choisis parmi les alcools, les alpha-diols, et les alkylphénols polyéthoxylés, polypropoxylés, ayant une chaîne grasse comportant par exemple 8 à 18 atomes de carbone, le nombre de groupements oxyde d'éthylène ou oxyde de propylène pouvant aller notamment de 2 à 50. On peut également citer les copolymères d'oxyde d'éthylène et de propylène, les condensats d'oxyde d'éthylène et de propylène sur des alcools gras ; les amides gras polyéthoxylés ayant de préférence de 2 à 30 moles d'oxyde d'éthylène, les amides gras polyglycérolés comportant en moyenne 1 à 5 groupements glycérol et en particulier 1,5 à 4 ; les amines grasses polyéthoxylées ayant de préférence 2 à 30 moles d'oxyde d'éthylène ; les esters d'acides gras du sorbitan oxyéthylénés ayant de 2 à 30 moles d'oxyde d'éthylène ; les esters d'acides gras du sucrose, les esters d'acides gras du polyéthylèneglycol, les alkylpolyglycosides, les dérivés de N-alkyl glucamine, les oxydes d'amines tels que les oxydes d'alkyl (C₁₀-C₁₄) amines ou les oxydes de N-acylaminopropylmorpholine. On notera que les alkylpolyglycosides constituent des tensio-actifs non-ioniques rentrant particulièrement bien dans le cadre de la présente invention tout comme les alcools gras éthoxylés.

Les agents tensioactifs amphotères ou zwitterioniques, dont la nature ne revet pas dans le cadre de la présente invention de caractère critique, peuvent être notamment des dérivés d'amines secondaires ou tertiaires aliphatiques, dans lesquels le radical aliphatique est une chaîne linéaire ou ramifiée comportant 8 à 18 atomes de carbone et contenant au moins un groupe anionique hydrosolubilisant, par exemple carboxylate, sulfonate, sulfate, phosphate ou phosphonate ; on peut citer encore les alkyl (C₈-C₂₀) bétaïnes, les sulfobétaïnes, les alkyl (C₈-C₂₀) amidoalkyl (C₁-C₆) betaïnes ou les alkyl (C₈-C₂₀) amidoalkyl (C₁-C₆) sulfobétaïnes.

Parmi les dérivés d'amines, on peut citer les produits vendus sous la dénomination MIRANOL, tels que décrits dans les brevets US-2 528 378 et US-2 781 354 et classés dans le dictionnaire CTFA, 3ème édition, 1982, sous les dénominations Amphocarboxyglycinates et Amphocarboxypropionates de structures respectives :

R₃₄-CONHCH₂CH₂-N(R₃₅)(R₃₆)(CH₂COO⁻)

dans laquelle : R₃₄ désigne un radical alkyle d'un acide R₃₄-COOH présent dans l'huile de coprah hydrolysée, un radical heptyle, nonyle ou undécyle, R₃₅ désigne un groupement bêta-hydroxyéthyle et R₃₆ un groupement carboxyméthyle ;
et

R₃₄'-CONHCH₂CH₂-N(B)(C)

dans laquelle :
B représente -CH₂CH₂OX', C représente -(CH₂)_{z} -Y', avec z = 1 ou 2,
X' désigne le groupement -CH₂CH₂-COOH ou un atome d'hydrogène
Y' désigne -COOH ou le radical -CH₂-CHOH-SO₃H
R₃₄' désigne un radical alkyle d'un acide R₃₇-COOH présent dans l'huile de coprah ou dans l'huile de lin hydrolysée, un radical alkyle, notamment en C₇, C₉, C₁₁ ou C₁₃, un radical alkyle en C₁₇ et sa forme iso, un radical C₁₇ insaturé.

Ces composés sont classés dans le dictionnaire CTFA, 5ème édition, 1993, sous les dénominations Disodium Cocoamphodiacetate, Disodium Lauroamphodiacetate, Disodium Caprylamphodiacetate, Disodium Capryloamphodiacetate, Disodium Cocoamphodipropionate, Disodium Lauroamphodipropionate, Disodium Caprylamphodipropionate, Disodium Capryloamphodipropionate, Lauroamphodipropionic acid, Cocoamphodipropionic acid.

A titre d'exemple on peut citer le cocoamphodiacetate commercialisé sous la dénomination commerciale MIRANOL® C2M concentré par la société RHODIA CHIMIE.

Parmi les tensioactifs cationiques on peut citer en particulier : les sels d'amines grasses primaires, secondaires ou tertiaires, éventuellement polyoxyalkylénées ; les sels d'ammonium quaternaire tels que les chlorures ou les bromures de tétraalkylammonium, d'alkylamidoalkyltrialkylammonium, de trialkylbenzylammonium, de trialkylhydroxyalkyl-ammonium ou d'alkylpyridinium; les dérivés d'imidazoline ; ou les oxydes d'amines à caractère cationique.

Les polyols selon l'invention sont des composés comportant de 2 à 100 atomes de carbone, de préférence de 3 à 50, et de 2 à 25 radicaux hydroxyle, de préférence de 2 à 10. Leur poids moléculaire est de préférence inférieur à 500. Parmi les polyols utilisables selon l'invention, on peut citer le propylène glycol, la glycérine, l'héxylène glycol, le butylène glycol, l'isopropène glycol, le néopentyl glycol, et les polyéthylène glycols.

Parmi les éthers de polyols utilisables selon l'invention, on peut citer le monométhyléther de propylène glycol et le monométhyléther de dipropylène glycol.

Les mono-alcools aromatiques utilisables selon l'invention comportent de 6 à 50 atomes de carbone. Le système cyclique aromatique peut être monocyclique ou polycyclique. Parmi les mono-alcools aromatiques selon l'invention, on peut citer l'alcool benzylique.

La composition conforme à l'invention peut en outre contenir un ou plusieurs colorants directs pouvant notamment être choisis parmi les colorants nitrés de la série benzénique, les colorants directs azoïques, les colorants directs méthiniques. Ces colorants directs peuvent être de nature non ionique, anionique ou cationique.

La composition de la présente invention peut en outre comprendre une ou plusieurs bases d'oxydation classiquement utilisées en coloration d'oxydation. A titre d'exemple, ces bases d'oxydation sont choisies parmi les para-phénylènediamines, les bis-phénylalkylènediamines, les para-aminophénols, les ortho-aminophénols, les bases hétérocycliques et leurs sels d'addition.

La composition de la présente invention peut en outre comprendre un ou plusieurs coupleurs classiquement utilisés en coloration d'oxydation. A titre d'exemple, ces coupleurs sont choisis parmi les méta-phénylènediamines, les méta-aminophénols, les méta-diphénols, les coupleurs naphtaléniques, les coupleurs hétérocycliques et leurs sels d'addition.

Le milieu approprié pour la teinture, appelé aussi support de teinture, est généralement constitué par de l'eau ou par un mélange d'eau et d'au moins un solvant organique pour solubiliser les composés qui ne seraient pas suffisamment solubles dans l'eau. A titre de solvant organique, on peut par exemple citer les alcanols inférieurs en C₁-C₄, tels que l'éthanol et l'isopropanol.

Les solvants sont, de préférence, présents dans des proportions de préférence comprises entre 1 et 40 % en poids environ par rapport au poids total de la composition tinctoriale, et encore plus préférentiellement entre 5 et 30 % en poids environ.

La composition conforme à l'invention peut également renfermer divers adjuvants utilisés classiquement dans les compositions pour la teinture des cheveux, tels que des polymères anioniques, cationiques, non-ioniques, amphotères, zwitterioniques ou leurs mélanges, des agents épaississants minéraux ou organiques, et en particulier les épaississants associatifs polymères anioniques, cationiques, non ioniques et amphotères, des agents antioxydants, des agents de pénétration, des agents séquestrants, des parfums, des tampons, des agents dispersants, des agents de conditionnement tels que par exemple des silicones volatiles ou non volatiles, modifiées ou non modifiées, des agents filmogènes, des céramides, des agents conservateurs, des agents opacifiants.

Les adjuvants ci-dessus sont en général présents en quantité comprise pour chacun d'eux entre 0,01 et 20 % en poids par rapport au poids de la composition.

Bien entendu, l'homme de l'art veillera à choisir ce ou ces éventuels composés complémentaires de manière telle que les propriétés avantageuses attachées intrinsèquement à la composition d'éclaircissement optique conforme à l'invention ne soient pas, ou substantiellement pas, altérées par la ou les adjonctions envisagées.

Le pH de la composition conforme à l'invention est généralement compris entre 4 et 12, et de préférence entre 5 et 11. Il peut être ajusté à la valeur désirée au moyen d'agents acidifiants ou alcalinisants habituellement utilisés en teinture des fibres kératiniques ou bien encore à l'aide de systèmes tampons classiques.

Parmi les agents acidifiants, on peut citer, à titre d'exemple, les acides minéraux ou organiques comme l'acide chlorhydrique, l'acide orthophosphorique, l'acide sulfurique, les acides carboxyliques comme l'acide acétique, l'acide tartrique, l'acide citrique, l'acide lactique, les acides sulfoniques.

Parmi les agents alcalinisants on peut citer, à titre d'exemple, l'ammoniaque, les carbonates alcalins, les alcanolamines telles que les mono-, di- et triéthanolamines ainsi que leurs dérivés, les hydroxydes de sodium ou de potassium et les composés de formule (V) suivante : 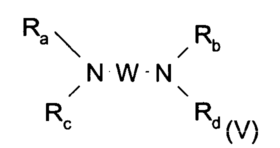 dans laquelle W est un reste propylène éventuellement substitué par un groupement hydroxyle ou un radical alkyle en C₁-C₄ ; Rₐ, R_{b}, R_{c} et R_{d}, identiques ou différents, représentent un atome d'hydrogène, un radical alkyle en C₁-C₄ ou hydroxyalkyle en C₁-C₄.

La composition selon l'invention peut se présenter sous des formes diverses, telles que sous forme de liquides, de crèmes, de gels, ou sous toute autre forme appropriée pour réaliser une teinture des fibres kératiniques, et notamment des cheveux humains.

Le procédé de teinture des fibres kératiniques de la présente invention consiste à appliquer une composition (a) comprenant, dans un milieu de teinture approprié, au moins un composé ortho ou α-dialdéhyde de formule (I) et une composition (b) comprenant, dans un milieu de teinture approprié, au moins un composé soufré de formules (II) ou (III) sur les fibres kératiniques pendant un temps de pose suffisant pour obtenir la coloration désirée.

Selon un mode de réalisation particulier, la composition (b) comprend de plus un ou plusieurs acides aminés.

Selon un autre mode de réalisation particulier, une composition (c) comprenant, dans un milieu de teinture approprié, un ou plusieurs acides aminés est de plus appliquée sur les fibres kératiniques.

Selon un autre mode de réalisation particulier, les compositions (a), (b) et / ou (c) comprennent de plus un composé choisi parmi les agents tensioactifs, les polyols, les éthers de polyols et les mono-alcools aromatiques.

Dans une première variante de ce procédé, les compositions (a), (b) et éventuellement (c) sont mélangées juste avant emploi et le mélange ainsi obtenu est appliqué sur les fibres kératiniques pendant un temps de pose suffisant pour obtenir la coloration désirée.

Dans une seconde variante de ce procédé, les compositions (a), (b) et éventuellement (c) sont appliquées successivement sur les fibres kératiniques pendant un temps de pose suffisant pour obtenir la coloration désirée, l'ordre d'application des différentes compositions étant indifférent.

Le temps de pose est généralement compris entre 5 minutes et 1 heure, de préférence entre 5 minutes et 30 minutes.

La température d'application est généralement fixée entre la température ambiante et 80°C, de préférence entre la température ambiante et 60 °C.

La présente invention a également pour objet un dispositif à plusieurs compartiments permettant de mettre en oeuvre le procédé de teinture des fibres kératiniques décrit ci-dessus.

Le dispositif à plusieurs compartiments de l'invention comprend un premier compartiment contenant une composition (a) comprenant, dans un milieu de teinture approprié, au moins un composé ortho ou α-dialdéhyde de formule (I) et un deuxième compartiment contenant une composition (b) comprenant, dans un milieu de teinture approprié, au moins un composé soufré de formules (II) ou (III).

Selon un mode de réalisation particulier du dispositif de l'invention, la composition (b) comprend de plus un ou plusieurs acides aminés.

Selon un autre mode de réalisation particulier du dispositif de l'invention, le dispositif comprend de plus un troisième compartiment contenant une composition (c) comprenant, dans un milieu de teinture approprié, un ou plusieurs acides aminés.

Selon un autre mode de réalisation particulier du dispositif de l'invention, les compositions (a), (b) et / ou (c) comprennent de plus un composé choisi parmi les agents tensioactifs, les polyols, les ethers de polyols et les mono-alcools aromatiques.

Ce dispositif peut être équipé d'un moyen permettant de délivrer sur les cheveux le mélange souhaité, tel que les dispositifs décrits dans le brevet FR-2 586 913 au nom de la demanderesse.

La présente invention a également pour objet l'utilisation pour la teinture et / ou l'éclaircissement optique des fibres kératiniques d'une composition comprenant au moins un composé ortho ou α-dialdéhyde de formule (I) et au moins un composé soufré de formules (II) ou (III).

Selon un mode de réalisation particulier, la composition utilisée comprend de plus un ou plusieurs acides aminés.

Selon un autre mode de réalisation particulier, la composition utilisée comprend de plus un composé choisi parmi les agents tensioactifs, les polyols, les éthers de polyols et les mono-alcools aromatiques.

Les exemples qui suivent servent à illustrer l'invention sans toutefois présenter un caractère limitatif.

### EXEMPLES

Dans les exemples qui suivent, les quantités des composés sont exprimées en grammes pour 100 g de composition (%) ou en moles pour 100 g de composition (moles %).

### Exemple 1

La composition 1 est réalisée comme ci-dessous :

| **Composés** | **Quantité** |
|---|---|
| Orthophthalaldéhyde | 0,5 % |
| Cystéamine dichlorhydrate | 3.10⁻³ mole % |
| Glycine | 10⁻³ Mole % |
| NaOH | q.s.p. pH 11 |
| Eau distillée | q.s.p. 100 g |

Cette composition est appliquée sur des mèches de cheveux gris à 90 % de cheveux blancs, naturels ou permanentés. Le rapport quantité de composition sur quantité de cheveu est égal à 5 et la température d'application est égale à la température ambiante. Après un temps de pose de 30 minutes, les mèches sont rincées, shampouinées et séchées.
La coloration obtenue est mesurée au moyen d'un spectrocolorimètre Minolta CM2002 (composantes spéculaires exclues, illuminant D65, angle 10°). Les résultats colorimétriques sont donnés dans le tableau ci-dessous :

| | **L*** | **a*** | **b*** | **Couleur** |
|---|---|---|---|---|
| Composition 1 ― cheveux naturels | 45,40 | 0,70 | 22,55 | Jaune |
| Composition 1 ― cheveux permanentés | 41,35 | 1,20 | 24,80 | Jaune |

Cette composition appliquée sur cheveu châtain conduit à un effet optique d'éclaircissement très esthétique.

### Exemple 2

La composition 2 est réalisée comme ci-dessous :

| **Composés** | **Quantité** |
|---|---|
| Orthophthalaldéhyde | 0,4 % |
| Cystéamine dichlorhydrate | 3,8 % |
| Eau distillée | q.s.p. 100 g |

Cette composition est appliquée sur des mèches de cheveux gris à 90 % de cheveux blancs, naturels ou permanentés. Le rapport quantité de composition sur quantité de cheveu est égal à 5 et la température d'application est égale à la température ambiante. Après un temps de pose de 15 minutes, les mèches sont rincées, shampouinées et séchées.
La coloration obtenue est mesurée au moyen d'un spectrocolorimètre Minolta CM2002 (composantes spéculaires exclues, illuminant D65, angle 10°). Les résultats colorimétriques sont donnés dans le tableau ci-dessous :

| | **L*** | **a*** | **b*** | **Couleur** |
|---|---|---|---|---|
| Composition 2 ― cheveux naturels | 47,00 | 4,15 | 20,80 | Jaune |
| Composition 2 ― cheveux permanentés | 46,35 | 4,10 | 22,70 | Jaune |

Dans le cas présent, il y a interaction de la composition avec les acides aminés présents dans le cheveu ce qui permet d'obtenir un éclaircissement optique sur cheveu châtains tenace, c'est-à-dire résistant aux shampooings et sans dégradation de la fibre kératinique.

### Exemples 3 à 7

Les compositions 3 à 7 sont réalisées comme ci-dessous :

| **Compositions** | **3** | **4** | **5** | **6** | **7** |
|---|---|---|---|---|---|
| Orthophthalaldéhyde | 0,5 % | 0,5 % | 0,4 % | 0,4 % | 0,4 % |
| Cystéamine | 0,034 % | 0,034 % | 0,013 % | 0,013 % | 0,013 % |
| Glycine | 0,0075 % | 0,0075 % | - | - | - |
| Alcool benzylique | 1 % | - | 1 % | - | - |
| Lauryléther sulfate de sodium (M.A.) | - | 1 % | - | 1 % | - |
| NaOH | q.s.p. pH 11 | q.s.p. pH 11 | - | - | - |
| Eau distillée | q.s.p. 100% | q.s.p. 100% | q.s.p. 100% | q.s.p. 100% | q.s.p. 100% |

Ces compositions sont appliquées sur des mèches de cheveux gris à 90 % de cheveux blancs, naturels ou permanentés. Le rapport quantité de composition sur quantité de cheveux est égal à 5 et la température d'application est égale à la température ambiante. Après un temps de pose de 30 minutes, les mèches sont rincées, shampouinées et séchées.
Les colorations obtenues sont mesurées au moyen d'un spectrocolorimètre Minolta CM3600d (composantes spéculaires inclues, illuminant D65, angle 10°). Les résultats colorimétriques sont donnés dans le tableau ci-dessous :

| | **L*** | **a*** | **b*** |
|---|---|---|---|
| Composition 3-cheveux naturels | 37,75 | 6,50 | 7,40 |
| Composition 3-cheveux permanentés | 27,30 | 7,85 | 5,35 |
| Composition 4-cheveux naturels | 40,30 | 5,30 | 6,30 |
| Composition 4-cheveux permanentés | 29,95 | 8,55 | 6,55 |
| Composition 5-cheveux naturels | 40,35 | 2,10 | 17,15 |
| Composition 5-cheveux permanentés | 39,00 | 4,65 | 17,65 |
| Composition 6-cheveux naturels | 51,70 | -1,70 | 13,30 |
| Composition 6-cheveux permanentés | 41,95 | 1,70 | 16,80 |
| Composition 7-cheveux naturels | 44,80 | 2,95 | 20,65 |
| Composition 7-cheveux permanentés | 40,95 | 4,00 | 17,60 |

### Exemples 8 à 12

Les compositions 8 à 12 sont réalisées comme ci-dessous :

| **Compositions** | **8** | **9** | **10** | **11** | **12** |
|---|---|---|---|---|---|
| Orthophthalaldéhyde | 0,5 % | 0,5 % | 0,4 % | 0,4 % | 0,4 % |
| Cystamine | 3.10⁻³ mole % | 3.10⁻³ mole % | 3.10⁻³ mole % | 3.10⁻³ mole % | 3.10⁻³ mole % |
| Glycine | 10⁻³ mole % | 10⁻³ mole % | 10⁻³ mole % | - | - |
| Alcool benzylique | 1 % | - | 1 % | - | - |
| Lauryléther sulfate de sodium (M.A.) | - | 1 % | - | 1 % | - |
| NaOH | q.s.p. pH 11 | q.s.p. pH 11 | - | - | - |
| Eau distillée | q.s.p. 100% | q.s.p. 100% | q.s.p. 100% | q.s.p. 100% | q.s.p. 100% |

Ces compositions sont appliquées sur des mèches de cheveux gris à 90 % de cheveux blancs, naturels ou permanentés. Le rapport quantité de composition sur quantité de cheveux est égal à 5 et la température d'application est égale à la température ambiante. Après un temps de pose de 30 minutes, les mèches sont rincées, shampouinées et séchées.
Les colorations obtenues sont mesurées au moyen d'un spectrocolorimètre Minolta CM3600d (composantes spéculaires inclues, illuminant D65, angle 10°). Les résultats colorimétriques sont donnés dans le tableau ci-dessous :

| | **L*** | **a*** | **b*** |
|---|---|---|---|
| Composition 8-cheveux naturels | 49,85 | 2,55 | 16,70 |
| Composition 8-cheveux permanentés | 50,00 | 4,05 | 20,45 |
| Composition 9-cheveux naturels | 57,00 | 0,90 | 15,35 |
| Composition 9-cheveux permanentés | 55,00 | 3,65 | 26,25 |
| Composition 10-cheveux naturels | 50,25 | 6,40 | 36,90 |
| Composition 10-cheveux permanentés | 52,90 | 2,05 | 22,80 |
| Composition 11-cheveux naturels | 43,65 | 6,30 | 22,10 |
| Composition 11-cheveux permanentés | 49,20 | 2,25 | 15,30 |
| Composition 12-cheveux naturels | 51,70 | 4,30 | 29,35 |
| Composition 12-cheveux permanentés | 51,00 | 3,50 | 28,35 |

## Revendications

1. Composition tinctoriale des fibres kératiniques comprenant, dans un milieu de teinture approprié :
- au moins un composé ortho ou α-dialdéhyde de formule (I) : 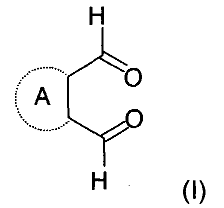
dans laquelle A est choisi parmi :
- un groupement mono ou polycarbocyclique, condensé ou non condensé, aromatique ou non aromatique, comprenant de 6 à 50 atomes de carbone ;
- un groupement mono ou polyhétérocyclique, condensé ou non condensé, aromatique ou non aromatique, comportant de 5 à 30 chaînons et contenant un ou plusieurs hétéroatomes choisis parmi l'azote, le soufre, l'oxygène et / ou le phosphore ;
le groupement A pouvant être substitué par des radicaux halogéno, alkyle en C₁-C₄, hydroxy, alcoxy en C₁-C₄, hydrogénocarbonyle, alkylcarbonyle en C₁-C₄, alkylthio en C₁-C₄, alkylcarboxy en C₁-C₄, nitro, sulfonato, ammonio, trialkylammonio en C₁-C₄, imidazolio, pyridinio, benzothiazolio ;
- au moins un composé soufré de formules (II) ou (III) :
R-SH (II)
R-S-S-R' (III)
dans lesquelles R et R', indépendamment l'un de l'autre, représentent un groupement comportant de 1 à 100 atomes de carbone, saturé ou insaturé, comportant éventuellement de 1 à 30 insaturations, ramifié ou non ramifié et pouvant comporter un ou plusieurs hétéroatomes choisis parmi l'azote, le soufre, l'oxygène et le phosphore, R et R' pouvant être substitués par un ou plusieurs radicaux choisis parmi les radicaux halogéno, alkyle en C₁-C₄, hydroxy, alcoxy en C₁-C₄, hydrogénocarbonyle, alkylcarbonyle en C₁-C₄, alkylthio en C₁-C₄, alkylcarboxy en C₁-C₄, amino, mono ou dialkylamino en C₁-C₄, mono ou dihydroxyalkylamino en C₁-C₄, nitro, sulfonato, ammonio, trialkylammonio en C₁-C₄, imidazolio, pyridinio, benzothiazolio.

2. Composition selon la revendication 1, dans laquelle le composé ortho ou α-dialdéhyde de formule (I) est un dérivé de l'orthophtalaldéhyde de formule (IV) : 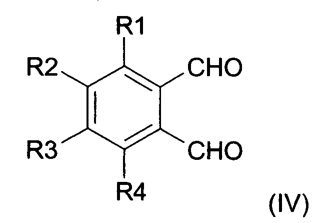 dans laquelle R₁, R₂, R₃ et R₄ représentent chacun indépendamment un atome d'hydrogène, un radical halogéno, alkyle en C₁₋₄, hydroxy, alcoxy en C₁₋₄, alkylthio en C₁-C₄ , alkylcarboxy en C₁-C₄, nitro, sulfonato ou un groupement hétérocyclique non aromatique azoté comportant de 5 à 8 chaînons.

3. Composition selon la revendication 2, dans laquelle les groupements R₁, R₂, R₃ et R₄ sont choisis parmi les groupements alkyle en C₁-C₄ et alcoxy en C₁-C₄.

4. Composition selon la revendication 3, dans laquelle le dérivé de l'orthophtalaldéhyde de formule (IV) est choisi parmi l'orthophtalaldéhyde et le 4,5-diméthoxyphtalaldéhyde.

5. Composition selon la revendication 1, dans laquelle le composé ortho ou α-dialdéhyde de formule (I) est choisi parmi les dérivés du naphtalènedicarboxaldéhyde, de l'anthracènedicarboxaldéhyde et du thiofènedicarboxaldéhyde.

6. Composition selon la revendication 5, dans laquelle le composé ortho ou α-dialdéhyde de formule (I) est choisi parmi le 1,2-naphtalènedicarboxaldéhyde, le 2,3-naphtalènedicarboxaldéhyde, le 2,3-anthracènedicarboxaldéhyde, le 2,3-thiofènedicarboxaldéhyde.

7. Composition selon l'une quelconque des revendications précédentes, dans laquelle R et R' comprennent de 1 à 4 atomes de carbone.

8. Composition selon la revendication 7, dans laquelle le composé soufré de formules (II) ou (III) est choisi parmi l'éthanethiol, le 2-aminothiophénol, l'acide thioglycolique, la cystéamine et la cystamine.

9. Composition selon l'une quelconque des revendications précédentes, dans laquelle la concentration en composé ortho ou α-dialdéhyde de formule (I) est comprise entre 0,01 et 30 % en poids rapporté au poids total de la composition.

10. Composition selon l'une quelconque des revendications précédentes, dans laquelle la concentration en composé soufré de formules (II) ou (III) est comprise entre 0,01 et 30 % en poids rapporté au poids total de la composition.

11. Composition selon l'une quelconque des revendications précédentes comprenant de plus un ou plusieurs acides aminés.

12. Composition selon la revendication 11, dans laquelle le ou les acides aminés sont choisis parmi la glycine, l'alanine, la valine, la leucine, l'isoleucine, la méthionine, la phénylalanine, la proline, la sérine, la thréonine, la cystéine, l'asparagine, la glutamine, la tyrosine, l'histidine, la lysine, l'ornithine, l'arginine, l'acide aspartique, l'acide glutamique, le tryptophane.

13. Composition selon la revendication 11 ou 12, dans laquelle la concentration en acides aminés est comprise entre 0,01 et 20 % en poids rapporté au poids total de la composition.

14. Composition selon l'une quelconque des revendications précédentes comprenant de plus un ou plusieurs peptides.

15. Composition selon l'une quelconque des revendications précédentes comprenant de plus une ou plusieurs protéines.

16. Composition selon l'une quelconque des revendications précédentes comprenant de plus un ou plusieurs agents tensioactifs.

17. Composition selon la revendication 16, dans laquelle le ou les agents tensioactifs sont de nature anionique, amphotère, non ionique, zwittérionique ou cationique.

18. Composition selon l'une quelconque des revendications précédentes comprenant de plus un ou plusieurs composés appartenant aux familles des polyols, des éthers de polyols et des mono-alcools aromatiques.

19. Composition selon la revendication 18, dans laquelle le ou les polyols sont des composés comportant de 2 à 100 atomes de carbone, de 2 à 25 radicaux hydroxyle et dont le poids moléculaire est inférieur à 500.

20. Composition selon la revendication 19, dans laquelle le ou les polyols sont choisis parmi le propylène glycol, la glycérine, l'héxylène glycol, le butylène glycol, l'isopropylène glycol, le néopentyl glycol et les polyéthylène glycols.

21. Composition selon la revendication 18, dans laquelle le ou les éthers de polyols sont choisis parmi le monométhyléther de propylène glycol et le monométhyléther de dipropylène glycol.

22. Composition selon la revendication 18, dans laquelle le ou les mono-alcools aromatiques sont des composés comportant de 6 à 50 atomes de carbone mono ou polycycliques.

23. Composition selon la revendication 22, dans laquelle le mono-alcool est l'alcool benzylique.

24. Composition selon l'une quelconque des revendications 16 à 23, dans laquelle la ou les concentrations en agents tensioactifs, polyols, éthers de polyols et mono-alcools aromatiques sont comprises entre 0,1 et 20 % en poids rapporté au poids total de la composition.

25. Procédé de teinture des fibres kératiniques, **caractérisé en ce qu'**une composition (a) comprenant, dans un milieu de teinture approprié, au moins un composé ortho ou α-dialdéhyde de formule (I) tel que défini dans l'une quelconque des revendications 1 à 6 et une composition (b) comprenant, dans un milieu de teinture approprié, au moins un composé soufré de formules (II) ou (III) tel que défini dans l'une quelconque des revendications 1, 7 et 8 sont appliquées sur les fibres kératiniques pendant un temps de pose suffisant pour obtenir la coloration désirée.

26. Procédé selon la revendication 25, dans lequel la composition (b) comprend de plus un ou plusieurs acides aminés tels que définis dans la revendication 11 ou 12.

27. Procédé selon la revendication 25 ou 26, dans lequel les compositions (a) et / ou (b) comprennent de plus un composé choisi parmi les agents tensioactifs, les polyols, les éthers de polyols et les mono-alcools aromatiques tels que définis dans l'une quelconque des revendications 16 à 23.

28. Procédé selon la revendication 25, dans lequel une composition (c) comprenant, dans un milieu de teinture approprié, un ou plusieurs acides aminés tels que définis dans la revendication 11 ou 12 est de plus appliquée sur les fibres kératiniques.

29. Procédé selon la revendication 28, dans lequel les compositions (a), (b) et / ou (c) comprennent de plus un composé choisi parmi les agents tensioactifs, les polyols, les éthers de polyols et les mono-alcools aromatiques tels que définis dans l'une quelconque des revendications 16 à 23.

30. Procédé selon l'une quelconque des revendications 25 à 27, dans lequel les compositions (a) et (b) sont mélangées juste avant emploi et le mélange ainsi obtenu est appliqué sur les fibres kératiniques pendant un temps de pose suffisant pour obtenir la coloration désirée.

31. Procédé selon la revendication 28 ou 29, dans lequel les compositions (a), (b) et (c) sont mélangées juste avant emploi et le mélange ainsi obtenu est appliqué sur les fibres kératiniques pendant un temps de pose suffisant pour obtenir la coloration désirée.

32. Procédé selon l'une quelconque des revendications 25 à 27, dans lequel les compositions (a) et (b) sont appliquées successivement sur les fibres kératiniques avec un éventuel rinçage intermédiaire, pendant un temps de pose suffisant pour obtenir la coloration désirée, l'ordre d'application des compositions (a) et (b) étant indifférent.

33. Procédé selon la revendication 28 ou 29, dans lequel les compositions (a), (b) et (c) sont appliquées successivement et éventuellement avec rinçage intermédiaire sur les fibres kératiniques pendant un temps de pose suffisant pour obtenir la coloration désirée, l'ordre d'application des compositions (a), (b) et (c) étant indifférent.

34. Dispositif à plusieurs compartiments, dans lequel un premier compartiment contient une composition (a) comprenant, dans un milieu de teinture approprié, au moins un composé ortho ou α-dialdéhyde de formule (I) tel que défini dans l'une quelconque des revendication 1 à 6 et un deuxième compartiment contient une composition (b) comprenant, dans un milieu de teinture approprié, au moins un composé soufré de formules (II) ou (III) tel que défini dans l'une quelconque des revendications 1, 7 et 8.

35. Dispositif selon la revendication 34, dans lequel la composition (b) comprend de plus un ou plusieurs acides aminés tels que définis dans la revendication 11 ou 12.

36. Dispositif selon la revendication 34 ou 35, dans lequel les compositions (a) et / ou (b) comprennent de plus un composé choisi parmi les agents tensioactifs, les polyols, les éthers de polyols et les mono-alcools aromatiques.

37. Dispositif selon la revendication 34 comprenant de plus un troisième compartiment contenant une composition (c) comprenant, dans un milieu de teinture approprié, un ou plusieurs acides aminés tels que définis dans la revendication 11 ou 12.

38. Dispositif selon la revendication 37, dans lequel les compositions (a), (b) et / ou (c) comprennent de plus un composé choisi parmi les agents tensioactifs, les polyols, les ethers de polyols et les mono-alcools aromatiques.

39. Utilisation pour la teinture des fibres kératiniques d'une composition comprenant, dans un milieu de teinture approprié, au moins un composé ortho ou α-dialdéhyde de formule (I) tel que défini dans l'une quelconque des revendications 1 à 6 et au moins un composé soufré de formules (II) ou (III) tel que défini dans l'une quelconque des revendications 1, 7 et 8.

40. Utilisation pour l'éclaircissement optique des fibres kératiniques d'une composition comprenant, dans un milieu de teinture approprié, au moins un composé ortho ou α-dialdéhyde de formule (I) tel que défini dans l'une quelconque des revendications 1 à 6 et au moins un composé soufré de formules (II) ou (III) tel que défini dans l'une quelconque des revendications 1, 7 et 8.

41. Utilisation selon la revendication 39 ou 40, dans laquelle la composition comprend de plus un ou plusieurs acides aminés tels que définis dans la revendication 11 ou 12.

42. Utilisation selon l'une quelconque des revendications 39 à 41, dans laquelle la composition comprend de plus un composé choisi parmi les agents tensioactifs, les polyols, les éthers de polyols et les mono-alcools aromatiques tels que définis dans l'une quelconque des revendications 16 à 23.
